# EUROPÄISCHE PATENTANMELDUNG

(11) **EP 2 428 247 A1**
(43) Veröffentlichungstag der Anmeldung: **14.03.2012**
(21) Anmeldenummer: 11180498.5
(22) Anmeldetag: 08.09.2011
(51) Int. Cl.: A61M 25/00, A61M 25/10, A61M 31/00

(54) **Harnröhrenkatheter zur Verankerung in der fossa navicularis der männlichen Harnröhre**

(30) Priorität: 10.09.2010 DE 102010040588
(71) Anmelder: Zenker, Ulrich, 85667 Grafing (DE)
(72) Erfinder: Zenker, Ulrich, 85667 Grafing (DE)
(74) Vertreter: Ruttensperger, Bernhard

(57) **Zusammenfassung**

Ein Harnröhrenkatheter zur Verankerung in der fossa navicularis umfasst einen Katheterkörper (12) mit einem in der Harnröhre (20) zu platzierenden Harnröhreneingriffsbereich (28) und einem in der fossa navicularis (30) zu positionierenden und zur Verankerung erweiterbaren Ballonbereich (34) im Harnröhreneingriffsbereich (28).

## Beschreibung

Die vorliegende Erfindung betrifft einen Harnröhrenkatheter.

Die bei männlichen Patienten eingesetzten Harnröhrenkatheter sind im Allgemeinen so aufgebaut, dass sie einen derart langen Katheterkörper aufweisen, dass ein Harnröhreneingriffsbereich desselben sich durch die gesamte Harnröhre hindurch erstreckend positioniert werden kann. Ein am distalen Endbereich des Katheterkörpers vorgesehener Ballonbereich liegt im Anwendungsfalle innerhalb der Harnblase, also außerhalb des Harnröhreneingriffsbereichs, und wird zum Verankern des Katheters durch Befüllung mit einem Fluid derart erweitert, dass er den Randbereich einer inneren Harnröhrenöffnung übergreift. Ein derartiger Harnröhrenkatheter weist im Allgemeinen also zwei in seinem inneren sich erstreckende Lumina auf. Ein erstes, im Allgemeinen größer dimensioniertes Lumen ist zum Inneren der Harnblase hin offen und ermöglicht somit das Entleeren/Befüllen der Harnblase durch den Katheterkörper hindurch. Ein zweites, im Allgemeinen etwas kleiner dimensioniertes Lumen dient zur Einleitung bzw. Ableitung des Fluids, welches zum Erweitern des Ballonbereichs dient.

Es ist die Aufgabe der vorliegenden Erfindung, einen Harnröhrenkatheter vorzusehen, welcher ein anderes Einsatzspektrum aufweist.

Erfindungsgemäß wird diese Aufgabe gelöst durch einen Harnröhrenkatheter zur Verankerung in der fossa navicularis, umfassend einen Katheterkörper mit einem in der Harnröhre zu platzierenden Harnröhreneingriffsbereich und einem in der fossa navicularis zu positionierenden und zur Verankerung erweiterbaren Ballonbereich im Harnröhreneingriffsbereich.

Der erfindungsgemäße Harnröhrenkatheter ist derart aufgebaut, dass der

Ballonbereich, welcher zur Verankerung des Harnröhrenkatheters in der Harnröhre dient, nicht in einem im Anwendungsfalle außerhalb der Harnröhre liegenden Bereich vorgesehen ist, sondern im Harnröhreneingriffsbereich liegt, also demjenigen Längenbereich des Katheterkörpers, welcher im Anwendungsfalle innerhalb der Harnröhre liegt.

Insbesondere ist der Ballonbereich derart am Harnröhreneingriffsbereich positioniert, dass er bei in der Harnröhre positioniertem Harnröhreneingriffsbereich in der fossa navicularis, also vergleichsweise nahe an der äußeren Harnröhrenöffnung liegt. Die fossa navicularis ist eine im Allgemeinen spindelartige Erweiterung der Harnröhre, welche in der glans penis kurz vor der äußeren Harnröhrenöffnung liegt. Dabei ist die Möglichkeit, eine Verankerung des Harnröhrenkatheters in der fossa navicularis vornehmen zu können, bei der vorliegenden Erfindung von besonderer Bedeutung. Wird der Ballon in einem Bereich der Harnröhre positioniert bzw. erweitert, welcher nicht im Bereich der fossa navicularis, sondern beispielsweise im Penisschaft liegt, so kann es auf Grund der Tatsache, dass die Harnröhre dort im Wesentlichen nicht erweiterbar ist, zu Harnröhrenverletzungen kommen.

Durch die Verankerung des Harnröhrenkatheters in diesem der äußeren Harnröhrenöffnung nahe liegenden Bereich der Harnröhre wird es möglich, auf einen durch die gesamte Harnröhre sich hindurch erstreckenden Abschnitt des Katheterkörpers zu verzichten. Dies wiederum gestattet es, einen so aufgebauten Harnröhrenkatheter beispielsweise bei der Durchführung eines Urethrocystogramms einzusetzen. Ein derartiges Urethrocystogramm dient dazu, vermittels eines in die Harnröhre applizierten Kontrastmittels durch Röntgenaufnahme Verengungen in der Harnröhre zu erkennen bzw. zu diagnostizieren. Bei der Durchführung dieser diagnostischen Maßnahme sollten die Harnröhre erweiternde und somit die Erkennbarkeit von Verengungen gefährdende Gegenstände, wie z.B. ein die gesamte Harnröhre durchsetzender Katheterkörper, nicht in der Harnröhre positioniert sein. Durch den Einsatz des erfindungsgemäßen, in der fossa navicularis zu verankernden Harnröhrenkatheters wird es jedoch möglich, einerseits das für die Durchführung eines Urethrocystogramms erforderliche Kontrastmittel zu applizieren, andererseits die Harnröhre zu verschließen, um das eingebrachte Kontrastmittel darin zu halten.

Um eine stabile Verankerung des Katheterkörpers in einer Harnröhre bei gleichzeitig nicht übermäßig großer Eindringlänge zu ermöglichen, wird weiter vorgeschlagen, dass der Harnröhreneingriffsbereich eine Länge von 2,5 bis 5,5 cm aufweist.

Weiter kann dazu vorgesehen sein, dass der Ballonbereich eine Länge von 1 bis 3 cm aufweist.

Da beim Positionieren des Katheterkörpers bzw. des Harnröhreneingriffsbereichs desselben in einer Harnröhre keine unmittelbare visuelle Kontrolle darüber vorliegt, ob der Ballonbereich im Erstreckungsbereich der fossa navicularis positioniert ist, wird weiter vorgeschlagen, dass ein Längenmittenbereich des Ballonbereichs zu einem unmittelbar außerhalb der Harnröhre einer äußeren Harnröhrenöffnung benachbart zu positionierenden Referenzbereich des Katheterkörpers einen Abstand im Bereich von 1 bis 3 cm aufweist.

Um eine das Einführen des Katheterkörpers einerseits ermöglichende, andererseits aber auch die stabile Verankerung gewährleistende Abstimmung auf die Querschnittsgröße der Harnröhre bzw. der fossa navicularis erreichen zu können, wird vorgeschlagen, dass der Katheterkörper eine Außenabmessung im Bereich von 0,4 bis 0,8 cm aufweist oder/und dass der Ballonbereich zur Verankerung in der fossa navicularis auf eine Außenabmessung im Bereich von 0,7 bis 2 cm erweiterbar ist.

Um eine visuelle Kontrolle des Ausmaßes, mit welchem der Katheterkörper in die Harnröhre eingeschoben ist, zu erleichtern, wird vorgeschlagen, dass im Referenzbereich wenigstens ein die Eindringtiefe des Katheterkörpers in eine Harnröhre anzeigender Indikator vorgesehen ist.

Dazu kann beispielsweise vorgesehen sein, dass der Indikator wenigstens eine Markierung am Katheterkörper umfasst.

Das Befüllen des Ballonbereichs mit Druckfluid kann beispielsweise dadurch ermöglicht werden, dass am Katheterkörper ein Anschlussbereich für eine Betätigungseinrichtung zum Erweitern des Ballonbereichs vorgesehen ist. An diesen Anschlussbereich kann beispielsweise eine herkömmliche und eine Flüssigkeit als Fluid enthaltende Spritze angeschlossen werden. Auf diese Art und Weise wird gleichzeitig eine Kontrolle des Volumens des in Richtung Ballonbereich abgegebenen Fluids gewährleistet.

Der Anschlussbereich kann beispielsweise im Referenzbereich vorgesehen sein und somit wenigstens einen Teil des Indikators bereitstellen, anhand welchem erkennbar wird, in welchem Ausmaß der Katheterkörper in die Harnröhre eingeführt worden ist.

Zur Manipulation des Harnröhrenkatheters, beispielsweise zum Strecken der Harnröhre bei Durchführung eines Urethrocystogramms, wird vorgeschlagen, dass am Katheterkörper eine Handgriffanordnung vorgesehen ist. Dabei kann beispielsweise vorgesehen sein, dass die Handgriffanordnung wenigstens ein ringartiges Eingriffselement umfasst.

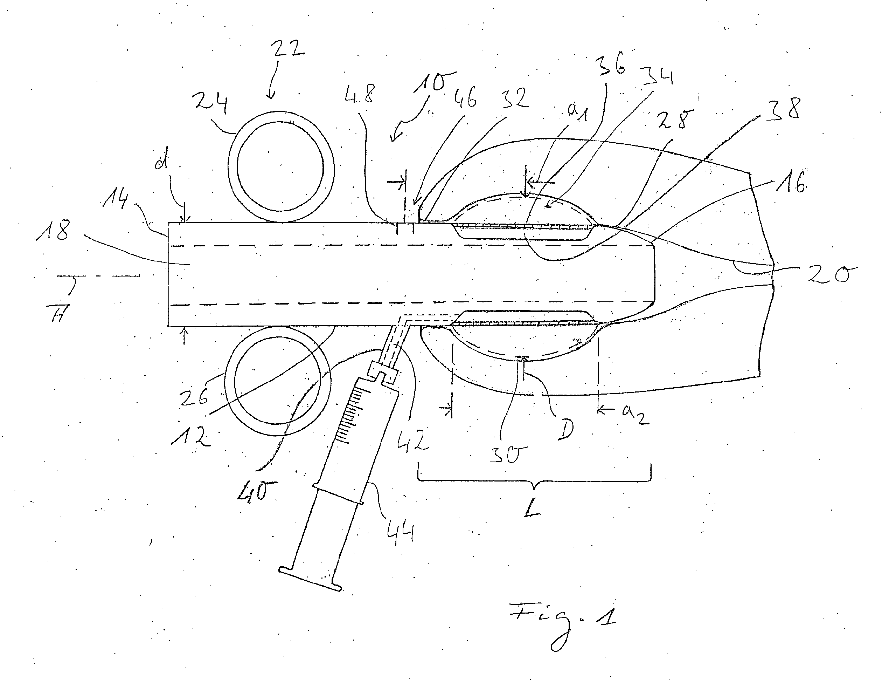

Die vorliegende Erfindung wird nachfolgend mit Bezug auf die beiliegende Fig. 1 detailliert beschrieben. Diese zeigt einen in eine Harnröhre eines männlichen Patienten eingeführten Harnröhrenkatheter in teilweise vereinfachter, teilweise geschnittener Darstellung.

Der in der Fig. 1 dargestellte Harnröhrenkatheter ist allgemein mit 10 bezeichnet. Dieser Harnröhrenkatheter 10 umfasst einen in einer Längsrichtung längs einer Längsmittenachse A langgestreckten Katheterkörper 12. Durch den Katheterkörper 12 erstreckt sich von einem proximalen Ende 14, welches auch im Anwendungsfalle außerhalb einer Harnröhre positioniert ist, zu einem distalen Ende 16, welches im Anwendungsfalle innerhalb einer Harnröhre liegt, ein durch Strichlinie angedeutetes Lumen 18. Dieses ist im Allgemeinen sowohl zum distalen Ende 16 als auch zum proximalen Ende 14 hin offen, um über den Harnröhrenkatheter 10 eine Verbindung zu einer Harnröhre 20 bzw. einer daran anschließenden Harnblase herstellen zu können. An das proximale Ende 14 können verschiedene Gerätschaften, wie z.B. ein Abschlussventil, eine Einrichtung zum Zuführen von in die Harnröhre 20 zu applizierender Flüssigkeit, beispielsweise Kontrastmittel, oder dergleichen angeschlossen werden.

Zur Manipulation des Harnröhrenkatheters 10 ist am Katheterkörper 12 in dessen außerhalb der Harnröhre 20 verbleibenden Bereich beispielsweise nahe dem proximalen Ende 14 eine Handgriffanordnung 22 vorgesehen. Diese umfasst zwei Eingriffsringe 24, 26, die nach Art einer Schere von der die Behandlung vornehmenden Person mit Daumen und Zeigefinger durchgriffen werden können.

Der in dem in Fig. 1 dargestellten Anwendungsfalle in der Harnröhre 20 zu positionierende Harnröhreneingriffsbereich 28 des Katheterkörpers 12 ist derart dimensioniert, dass er sich über die fossa navicularis 30 hinaus erstreckt. Dazu weist der Harnröhreneingriffsbereich 28 eine Länge L im Bereich von 1 bis 5cm auf. Dies bedeutet, dass das distale Ende 16 in der Harnröhre 20 an der von einer äußeren Harnröhrenöffnung 32 abgewandten Seite der fossa navicularis 30 zu liegen kommt.

Im Harnröhreneingriffsbereich 28 weist der Harnröhrenkatheter 10 einen allgemein mit 34 bezeichneten Ballonbereich auf. Dieser umfasst eine von einem am Katheterkörper 12 sich beispielsweise ringartig um die Längsmittenachse desselben erstreckenden Membranbereich 36 umschlossene Kammer 38 mit veränderbarem Volumen. Durch Zufuhr von Fluid, beispielsweise unter Druck stehender Flüssigkeit, zu der Kammer 38 wird der Membranbereich 36 ausgedehnt und kann beispielsweise in die in der Fig. 1 mit Strichlinie eingezeichnete erweiterte Konfiguration gebracht werden. Liegt dabei der Ballonbereich innerhalb der fossa navicularis, führt das Erweitern des Membranbereichs 36 zur Verankerung des Harnröhrenkatheters 10 in der Harnröhre 20 durch einen in Längsrichtung des Katheterkörpers 12 generierten Formschlusseingriff zwischen dem erweiterten Ballonbereich 34 und der fossa navicularis.

Um der Kammer 38 das unter Druck stehende Fluid zuführen zu können, ist am Katheterkörper 12 ein von diesem beispielsweise seitlich bzw. quer zur Längsmittenachse A abzweigender Anschlussbereich 40 vorgesehen. Durch den Anschlussbereich 40 und einen Abschnitt des Katheterkörpers 12 erstreckt sich ein Lumen 42, das zur Kammer 38 führt und die Zufuhr bzw. Abfuhr von Fluid zu bzw. aus dieser ermöglicht. Am freien Endbereich kann der Anschlussbereich 40 mit einer Ventilanordnung, beispielsweise einem Rückschlagventil, ausgebildet sein, welches durch das Einführen einer beispielsweise als Spritze ausgebildeten Betätigungseinrichtung 44 geöffnet werden kann. Durch diese Spritze bzw. Betätigungseinrichtung 44 kann ein definiertes Volumen von Fluid über das Lumen 42 in die Kammer 38 abgegeben werden, so dass in entsprechend definiertem Ausmaß die gewünschte Erweiterung des Membranbereichs 36 hervorgerufen werden kann. Nach erfolgter Erweiterung kann die Spritze bzw. Betätigungseinrichtung 44 vom Anschlussbereich 40 entfernt werden, was das Handhaben des Harnröhrenkatheters 10 erleichtert. Auf Grund der vorangehend angesprochenen Ventilanordnung wird das ungewünschte Entweichen von den Membranbereich 36 in seinem erweiterndem Zustand haltendem Fluid verhindert werden.

In einem im Anwendungsfalle unmittelbar außerhalb der Harnröhre 20, also benachbart der äußeren Harnröhrenöffnung 32 positionierten bzw. zu positionierenden Referenzbereich 46 ist zumindest ein das Ausmaß des Einführens des Katheterkörpers 12 in die Harnröhre 20 anzeigender Indikator vorgesehen. Dieser Indikator kann eine oder mehrere Markierungen 48 an der Oberfläche des Katheterkörpers 12 umfassen. Diese Markierungen 48 sind bezüglich des Ballonbereichs 34 derart am Katheterkörper 12 positioniert, dass dann, wenn diese Markierungen 48 der äußeren Harnröhrenöffnung 32 unmittelbar benachbart liegen, also noch sichtbar sind, sichergestellt ist, dass der Ballonbereich 34 in demjenigen Längenbereich der Harnröhre 20 liegt, in welchem die fossa navicularis 30 sich erstreckt. Alternativ oder zusätzlich kann als Indikator der bei dem in der Fig. 1 dargestellten Harnröhrenkatheter 10 ebenfalls im Referenzbereich 46 vorgesehene Anschlussbereich 40 dienen. Auch dessen Positionierung unmittelbar benachbart der äußeren Harnröhrenöffnung 32 zeigt an, dass der Ballonbereich 34 mit der fossa navicularis 30 ausgerichtet ist und durch Zuführen von Fluid zur Kammer 38 und damit Erweitern des Membranbereichs 36 der Harnröhrenkatheter 10 verankert werden kann.

Da bei der Einbringung des Katheterkörpers 12 in die Harnröhre 20 keine unmittelbare visuelle Kontrolle der Positionierung des Ballonbereichs 34 in der fossa navicularis 30 möglich ist, ist es vorteilhaft, unter Berücksichtigung der allgemein bekannten Anatomie der Harnröhre 20 bzw. der fossa navicularis 30 den Harnröhrenkatheter 10 mit darauf abgestimmten Dimensionierungen bereitzustellen. So sollte beispielsweise die Längserstreckung a₂ des Ballonbereichs 34 auf die Längserstreckung der fossa navicularis abgestimmt sein und in einem Bereich von 1 bis 3 cm liegen. Der Abstand a₁ eines Längenmittenbereichs des Ballonbereichs 34 zum Referenzbereich 46 liegt vorzugsweise im Bereich von 1 bis 2 cm.

Die Außenabmessung, also beispielsweise der Außendurchmesser d, des Katheterkörpers 12 sollte insbesondere in dem innerhalb der Harnröhre 20 zu positionierenden Harnröhreneingriffsbereich 28 im Bereich von 0,4 bis 0,8 cm liegen. Der Ballonbereich 34 bzw. der Membranbereich 36 desselben sollte so ausgebildet sein, dass er im verankerten Zustand eine Außenabmessung, also beispielsweise Außendurchmesser D, im Bereich von 0,7 bis 2 cm aufweist.

Mit dem erfindungsgemäßen Aufbau eines Harnröhrenkatheters 10 wird es möglich, das Einsatzspektrum eines derartigen Katheters zu erweitern. Insbesondere wird dessen Anwendung beispielsweise bei Durchführung eines Urethrocystogramms ermöglicht. Hierzu wird der Harnröhrenkatheter 10 in der in Fig. 1 dargestellten Art und Weise in der Harnröhre 20 bzw. der fossa navicularis 30 verankert. Daraufhin kann durch das Lumen 18 im Katheterkörper 12 hindurch ein Kontrastmittel in die Harnröhre 20 eingespritzt werden. Durch das Ergreifen an der Handgriffanordnung 22 wird es möglich, mit dem in der fossa navicularis 30 verankerten Harnröhrenkatheter 10 die Harnröhre 20 bei Erzeugung einer Röntgenaufnahme zu strecken, um auf diese Art und Weise Verengungen in der Harnröhre 20 sichtbar zu machen. Auch andere Anwendungsmöglichkeiten, wie z.B. die Okklusion der Harnröhre zur Verhinderung einer Harninkontinenz, sind möglich.

Der in der Fig. 1 dargestellte Harnröhrenkatheter 10 kann in einfacher Weise aus Kunststoff und als Wegwerfteil bereitgestellt werden. Dies vermeidet Probleme bei der Sterilisierung und vermindert somit die Infektionsgefahr.

Ein Harnröhrenkatheter zur Verankerung in der fossa navicularis umfasst einen Katheterkörper (12) mit einem in der Harnröhre (20) zu platzierenden Harnröhreneingriffsbereich (28) und einem in der fossa navicularis (30) zu positionierenden und zur Verankerung erweiterbaren Ballonbereich (34) im Harnröhreneingriffsbereich (28).

## Patentansprüche

1. Harnröhrenkatheter zur Verankerung in der fossa navicularis, umfassend einen Katheterkörper (12) mit einem in der Harnröhre (20) zu platzierenden Harnröhreneingriffsbereich (28) und einem in der fossa navicularis (30) zu positionierenden und zur Verankerung erweiterbaren Ballonbereich (34) im Harnröhreneingriffsbereich (28).

2. Harnröhrenkatheter nach Anspruch 1,
**dadurch gekennzeichnet, dass** der Harnröhreneingriffsbereich (28) eine Länge von 2,5 bis 5,5 cm aufweist.

3. Harnröhrenkatheter nach Anspruch 1 oder 2,
**dadurch gekennzeichnet, dass** der Ballonbereich (34) eine Länge (a₂) von 1 bis 3 cm aufweist.

4. Harnröhrenkatheter nach einem der Ansprüche 1 bis 3,
**dadurch gekennzeichnet, dass** ein Längenmittenbereich des Ballonbereichs (34) zu einem unmittelbar außerhalb der Harnröhre (20) einer äußeren Harnröhrenöffnung (32) benachbart zu positionierenden Referenzbereich (46) des Katheterkörpers (12) einen Abstand (a₁) im Bereich von 1 bis 3cm aufweist.

5. Harnröhrenkatheter nach einem der Ansprüche 1 bis 4,
**dadurch gekennzeichnet, dass** der Katheterkörper (12) eine Außenabmessung (d) im Bereich von 0,4 bis 0,8 cm aufweist oder/und dass der Ballonbereich (34) zur Verankerung in der fossa navicularis auf eine Außenabmessung (D) im Bereich von 0,7 bis 2 cm erweiterbar ist.

6. Harnröhrenkatheter nach Anspruch 4 oder Anspruch 5, sofern auf Anspruch 4 rückbezogen,
**dadurch gekennzeichnet, dass** im Referenzbereich (46) wenigstens ein die Eindringtiefe des Katheterkörpers (12) in eine Harnröhre (20) anzeigender Indikator vorgesehen ist.

7. Harnröhrenkatheter nach Anspruch 6,
**dadurch gekennzeichnet, dass** der Indikator wenigstens eine Markierung (48) am Katheterkörper (12) umfasst.

8. Harnröhrenkatheter nach einem der Ansprüche 1 bis 7,
**dadurch gekennzeichnet, dass** am Katheterkörper (12) ein Anschlussbereich (40) für eine Betätigungseinrichtung (44) zum Erweitern des Ballonbereichs (34) vorgesehen ist.

9. Harnröhrenkatheter nach Anspruch 8 und Anspruch 6 oder 7,
**dadurch gekennzeichnet, dass** der Anschlussbereich (40) im Referenzbereich (46) vorgesehen ist und wenigstens einen Teil des Indikators bildet.

10. Harnröhrenkörper nach einem der Ansprüche 1 bis 9,
**dadurch gekennzeichnet, dass** am Katheterkörper (12) eine Handgriffanordnung (22) vorgesehen ist.

11. Harnröhrenkatheter nach Anspruch 10,
**dadurch gekennzeichnet, dass** die Handgriffanordnung (22) wenigstens ein ringartiges Eingriffselement (24, 26) umfasst.
